# EUROPÄISCHE PATENTSCHRIFT

(11) **EP 3 154 509 B1**
(45) Veröffentlichungstag und Bekanntmachung des Hinweises auf die Patenterteilung: **04.07.2018**
(21) Anmeldenummer: 15725349.3
(22) Anmeldetag: 29.05.2015
(51) Int. Cl.: A61K 8/67, A61Q 15/00, A61Q 19/02, A61K 8/97

(54) **KOSMETISCHE ZUSAMMENSETZUNGEN ZUR HAUTAUFHELLUNG**
COSMETIC COMPOSITIONS FOR BLEACHING THE SKIN
COMPOSITIONS COSMÉTIQUES SERVANT À ÉCLAIRCIR LA PEAU

(30) Priorität: 11.06.2014 DE 102014211185
(43) Veröffentlichungstag der Anmeldung: 19.04.2017
(73) Patentinhaber: Henkel AG & Co. KGaA, 40589 Düsseldorf (DE)
(72) Erfinder: DÖRING, Thomas, 41540 Dormagen (DE); SCHEVARDO, Natascha, 40699 Erkrath (DE); GIESEN, Melanie, 47608 Geldern (DE)
(86) Internationale Anmeldenummer: PCT/EP2015/061954
(87) Internationale Veröffentlichungsnummer: WO 2015/189047

(56) Entgegenhaltungen:
- WO-A1-99/55352
- WO-A2-01/91715
- DE-A1- 4 227 806
- DE-A1-102005 031 482
- US-A- 5 609 875
- US-A1- 2006 216 254
- DATABASE GNPD [Online] MINTEL; Februar 2006 (2006-02), "Spot Treatment Créme", XP002742498, Database accession no. 429359
- DATABASE GNPD [Online] MINTEL; Oktober 2013 (2013-10), "Night Cream", XP002742499, Database accession no. 2137612
- DATABASE GNPD [Online] MINTEL; Juni 2012 (2012-06), "BB Cream", XP002742500, Database accession no. 1811286
- DATABASE GNPD [Online] MINTEL; Dezember 2004 (2004-12), "Whitening Night Cream", XP002742501, Database accession no. 324341

## Beschreibung

Die Erfindung liegt auf dem Gebiet der Kosmetik und betrifft kosmetische Antitranspirant-Zusammensetzungen zur Hautaufhellung, die mindestens einen öligen Pflanzenextrakt aus der Gattung der Süßhölzer und mindestens einen Ascorbylsäureester enthalten.

Die Erfindung betrifft weiterhin die kosmetische, nicht-therapeutische Verwendung der kosmetischen Zusammensetzungen zur Behandlung von Überpigmentierungen, Pigmentstörungen, und/oder postinflammatorischen Hyperpigmentierungen im axillaren Bereich der Haut, sowie ein Verfahren zur Herstellung kosmetischer Hautaufhellungs-Zusammensetzungen in Form von Emulsionen, die einen öligen Pflanzenextrakt aus der Gattung der Süßhölzer und mindestens einen Ascorbylsäureester enthalten.

Die Pigmentierung der Haut erfolgt in den Melanozyten, welche in der untersten Schicht der Epidermis neben den Basalzellen als je nach Hauttyp entweder vereinzelt oder gehäuft auftretende pigmentbildende Zellen vorzufinden sind. Die Melanozyten bilden Melanosomen, in denen wiederum Melanin gebildet wird. Durch verschiedene chemische und/oder physikalische Einflüsse, insbesondere durch UV-Strahlung, wird verstärkt Melanin gebildet. Dieses wird über die Keratinozyten in die Corneozyten (Hornschicht) transportiert und führt zu einer bräunlichen bis braun-schwarzen Hautfarbe. Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird.

Die Melaninbildung - und damit Haut- und Haarfarbe - unterliegt äußeren Einflüssen und kann neben erwünschten Effekten ("gesunde Bräune") auch zu unerwünschten Erscheinungen führen. So kann z.B. UV-Strahlung zu Sommersprossen führen. Fehlpigmentierungen können auch aufgrund genetischer Disposition, Wundheilung bzw. -vernarbung oder Hautalterung ("Altersflecken") auftreten.

Auch hormonell bedingte Störungen können für abnormale Reaktionen der Melanozyten und dadurch zu einer verstärkten Anreicherung von Melanin in der Haut verantwortlich sein, wodurch unerwünschte braune Flecken auf der Haut entstehen.

Es ist daher wünschenswert, kosmetische Zusammensetzungen zur Verfügung zu haben, mit denen jedwede "Fehlpigmentierung" der Haut nachhaltig und wirksam verhindert, vermindert und/oder beseitigt werden kann.

Aus dem Stand der Technik ist eine Vielzahl hautaufhellender Zusammensetzungen bekannt.

Auf weißen Pigmenten basierende Zusammensetzungen weisen den Nachteil auf, dass der Hautaufhellungseffekt nicht lange anhält und oftmals unnatürlich wirkt. Andere Wirkstoffe - wie beispielsweise Reduktionsmittel mit Hydrochinon- oder Resorcin-Struktureinheiten - rufen hervorragende Hautaufhellungseffekte hervor, doch sind sie schlecht hautverträglich und wirken teilweise toxisch auf die Melanocyten. Dauerhafte Pigmentveränderungen können die Folgen sein.

In DE 102005031482 werden Hautaufhellungsmittel vorgeschlagen, die Ascorbinsäure oder deren physiologisch verträglichen Derivate oder Salze sowie 8-Hexadecen-1,16-dicarbonsäure, Sebacinsäure und/oder Azelainsäure enthalten. Als fakultativen Wirkstoff können die Hautaufhellungsmittel weiterhin wässrig-alkoholische Licorice-Extrakte enthalten.

In DE 4227806 werden kosmetische Zusammensetzungen zur Aufhellung von Hautflecken offenbart, die ein Flavonoid (beispielsweise aus Lakritzenextrakt) und ein DOPA-Chinon-Reduktionsmittel (beispielsweise Ascorbinsäure oder bestimmte Ascorbylester) enthalten.

Beide Zusammensetzungen beruhen auf wässrigen und/oder wässrig-alkoholischen Licorice-Extrakten oder auf wasserlöslichen Flavonoiden in Kombination mit Ascorbinsäure(derivaten).

Untersuchungen mit unterschiedlichen Pflanzenextrakten ergaben, dass ölige Süßholzextrakte den wässrigen oder wässrig-alkoholischen Süßholzextrakten in Bezug auf deren hautaufhellende Wirkung weit überlegen sind, weshalb es erstrebenswert ist, in kosmetischen Hautaufhellungszusammensetzungen ölige Süßholzextrakte zu verwenden.

WO0191715 und US2006216254 offenbaren Kombinationen aus öllöslichen Licorice-Wurzeln-Extrakt und Ascorbylsäureester in hautaufhellende Zusammensetzungen. Bei der Einarbeitung öliger Süßholzextrakte in kosmetische Emulsionen - der bevorzugten Applikationsform für Hautpflegemittel - zeigten diese jedoch eine unzureichende Löslichkeit in der Wasserphase, wodurch sowohl die Optik (starke Trübung der Wasserphase) als auch die Stabilität der Zusammensetzungen beeinträchtigt wurde. Dieses Problem konnte auch durch Zugabe von Lösungsvermittlern nicht behoben werden.

Aufgabe der vorliegenden Erfindung war es ölige Süßholzextrakte in kosmetische Zusammensetzungen für die topische Applikation einzuarbeiten und darin zu stabilisieren. Die Zusammensetzungen sollten einen hautaufhellenden Effekt auf über- oder fehlpigmentierter Haut hervorrufen und sehr gut hautverträglich sein.

Vollkommen überraschend wurde gefunden, dass die zuvor genannten Aufgaben durch Kombination eines öligen Süßholzextraktes mit mindestens einem Ascorbylsäureester gelöst werden können.

Ein erster Gegenstand der Erfindung ist daher eine kosmetische Antitranspirant-Zusammensetzung zur Hautaufhellung im axillaren Bereich, die
a) mindestens einen öligen Pflanzenextrakt aus der Gattung der Süßhölzer (*Glycyrrhiza*) und
b) mindestens einen Ascorbylsäureester und
c) mindestens einen Antitranspirant-Wirkstoff, enthält.

Die erfindungsgemäßen Zusammensetzungen weisen eine Reihe von Vorteilen auf:
- sie sind einfach herstellbar und lassen sich selbst als Emulsion problemlos formulieren, ohne dass Trübungs-, Separations- und/oder Stabilitätsprobleme auftreten,
- durch Zugabe des Ascorbylsäureesters können ölige Süßholzextrakte in der Wasserphase klar gelöst werden, wodurch sowohl optisch als auch anwendungstechnisch ansprechende Produkte erhalten werden können,
- die Zusammensetzungen weisen eine hervorragende hautaufhellende Wirkung auf und eignen sich zur Vorbeugung und/oder für die Behandlung von Überpigmentierungen, Pigmentierungsstörungen, Altersflecken und/oder postinflammatorischen Hyperpigmentierungen der Haut,
- die Wirkstoffkombination aus einem öligen Süßholzextrakt und einem Ascorbylsäureester wirkt synergistisch in Bezug auf die Hautaufhellung,
- die Zusammensetzungen sind sehr gut hautverträglich,
- gute Bioverfügbarkeit der Wirkstoffe.

Unter einem "öligen" Extrakt wird erfindungsgemäß verstanden, dass der Süßholzextrakt in einer bei Raumtemperatur wasserunlöslichen, vorzugsweise flüssigen, organischen Verbindung gelöst vorliegt.
Unter "wasserunlöslich" wird verstanden, dass sich bei Raumtemperatur maximal 1 g, vorzugsweise maximal 0,5 g, mehr bevorzugt maximal 0,1 g und insbesondere bevorzugt maximal 0,01 g der organischen Verbindung in 1 l Wasser lösen.
Unter "organischen Verbindungen" sind vorzugsweise synthetische, mineralische oder natürliche Öle (Triglyceride) zu verstehen, die an späterer Stelle der Anmeldung näher beschrieben werden. Unter "Hautaufhellung" wird die Veränderung der Hautfarbe, bedingt durch eine verminderte Neubildung von Melanin, verstanden.

Der ölige Pflanzenextrakt aus der Gattung der Süßhölzer (*Glycyrrhiza*) kann prinzipiell aus allen Teilen wie den Blättern, den Blüten, den Samen und/oder den Wurzeln von Süßhölzern hergestellt werden.
Besonders geeignet im Sinne der vorliegenden Erfindung sind jedoch die öligen Extrakte aus den Wurzeln von *Glycyrrhiza Glabra* (Licorice-Wurzeln), da sie den stärksten synergistischen Hautaufhellungseffekt in Verbindung mit dem Ascorbylsäureester zeigen.

In einer ersten bevorzugten Ausführungsform enthalten die kosmetischen Zusammensetzungen zur Hautaufhellung daher einen öligen Extrakt aus den Wurzeln von *Glycyrrhiza Glabra* (Licorice-Wurzeln).

Als geeignetes öliges Extraktionsmedium für die Licorice-Wurzeln kommen prinzipiell alle physiologisch verträglichen, synthetischen, mineralischen oder natürlichen Öle in Frage. Bevorzugte Extraktionsmittel sind jedoch die sogenannten Esteröle.
Unter Esterölen sind die Ester von geradkettigen oder verzweigten, gesättigten oder ungesättigten C₆-C₃₀-Carbonsäuren mit geradkettigen oder verzweigten, gesättigten oder ungesättigten C₂-C₃₀-Alkoholen zu verstehen. Bevorzugt sind die Monoester gesättigter oder ungesättigter C₈-C₂₄-Carbonsäuren mit geradkettigen oder verzweigten Alkoholen mit 2 bis 24 C-Atomen.
Besonders bevorzugt sind Monoester gesättigter oder ungesättigter C₁₀-C₂₂-Carbonsäuren mit geradkettigen oder verzweigten - vorzugsweise verzweigten - C₃-C₁₀-Alkoholen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate, Laurinsäurehexylester (Cetiol® A), Di-n-butyladipat (Cetiol® B), Myristylmyristat (Cetiol® MM), Cetearyl Isononanoate (Cetiol® SN) und Ölsäuredecylester (Cetiol® V).
Ganz besonders bevorzugt sind Isopropylmyristat, Isopropylpalmitat und 2-Ethylhexylpalmitat; insbesondere bevorzugt ist Isopropylmyristat.

In einer zweiten bevorzugten Ausführungsform enthalten die kosmetischen Zusammensetzungen zur Hautaufhellung einen Isopropylmyristat-Extrakt aus den Wurzeln von *Glycyrrhiza Glabra* (Licorice-Wurzeln).

Ein für die erfindungsgemäßen Zusammensetzungen geeigneter öliger Pflanzenextrakt aus der Gattung der Süßhölzer (*Glycyrrhiza*) ist beispielsweise der unter der Handelsbezeichnung "Liquorice Herbasol® Extract IPM" von der Firma Cosmetochem erhältlich.

Die öligen Pflanzenextrakte aus der Gattung der Süßhölzer enthalten bevorzugt einen Süßholz-Wirkstoffgehalt von 0,001 bis 90 Gew.-%, mehr bevorzugt von 0,002 bis 70 Gew.-%, besonders bevorzugt von 0,005 bis 50 Gew.-%, ganz besonders bevorzugt von 0,01 bis 30 Gew.-% und insbesondere von 0,05 bis 10 Gew.-%, wobei sich die Mengenangaben auf das Gesamtgewicht des öligen Pflanzenextraktes beziehen.

Die kosmetischen Zusammensetzungen enthalten den öligen Pflanzenextrakt aus der Gattung der Süßhölzer bevorzugt in einer Menge von 0,01 bis 5 Gew.-%, mehr bevorzugt von 0,05 bis 4 Gew.-%, besonders bevorzugt von 0,1 bis 3 Gew.-% und insbesondere von 0,2 bis 2 Gew.-%, wobei sich die Mengenangaben auf das Gesamtgewicht der kosmetischen Zusammensetzung beziehen. Gemäß einer bevorzugten Ausführungsform werden in den erfindungsgemäßen Zusammensetzungen 0,01 bis 5 Gew.-%, mehr bevorzugt von 0,05 bis 4 Gew.-%, besonders bevorzugt von 0,1 bis 3 Gew.-% und insbesondere von 0,2 bis 2 Gew.-% Liquorice Herbasol® Extract IPM von der Firma Cosmetochem eingesetzt; die Mengenangaben beziehen sich dabei auf das Gesamtgewicht der kosmetischen Zusammensetzungen.

In einer dritten bevorzugten Ausführungsform enthalten die erfindungsgemäßen kosmetischen Zusammensetzungen einen Ascorbylsäureester, der ausgewählt ist aus Verbindungen der folgenden Formel (I), 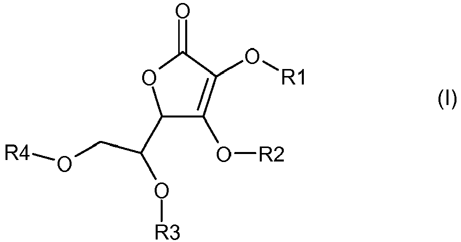 in der
einer bis vier der Reste R1 bis R4 für die Gruppierung -C(O)-R und die anderen Reste ggfs. für Wasserstoff stehen; und R für einen geradkettigen oder verzweigten, gesättigten oder ungesättigten Alk(en)ylrest mit 8 bis 24, bevorzugt mit 10 bis 20 und insbesondere mit 13 bis 17 Kohlenstoffatomen steht.

Es wurde gefunden, dass sie synergistische Hautaufhellungswirkung verstärkt werden kann, wenn bestimmte Ascorbylsäureester in Kombination mit öligen Süßholzextrakten verwendet werden. Besonders bevorzugte Ester nach Formel (I) sind ausgewählt aus Ascorbylpalmitat, Ascorbylisopalmitat, Ascorbyltetraisopalmitat, Ascorbylstearat, Ascorbylisostearat, Ascorbyloleat, und/oder Ascorbyllinoleat.
Insbesondere bevorzugt ist Ascorbyltetraisopalmitat, denn die Kombination von Ascorbyltetraisopalmitat mit öligen Süßholzextrakten führte zu der stärksten synergistischen Hautaufhellungswirkung. Darüber hinaus lösten sich die öligen Süßholzextrakte in der Wasserphase von Emulsionen besonders gut, wenn der Wasserphase Ascorbyltetraisopalmitat hinzugefügt wurde.

In einer weiteren bevorzugten Ausführungsform enthalten die kosmetischen Zusammensetzungen daher als Ascorbylsäureester nach Formel (I) mindestens eine Verbindung, ausgewählt aus Ascorbylpalmitat, Ascorbylisopalmitat, Ascorbyltetraisopalmitat, Ascorbylstearat, Ascorbylisostearat, Ascorbyloleat, und/oder Ascorbyllinoleat.

In einer insbesondere bevorzugten Ausführungsform enthalten die kosmetischen Zusammensetzungen Ascorbyltetraisopalmitat.

Der mindestens eine Ascorbylsäureester wird in den kosmetischen Zusammensetzungen bevorzugt in einem Gewichtsanteil von 0,005 bis 1 Gew.-% am Gesamtgewicht der kosmetischen Zusammensetzung eingesetzt, besonders bevorzugt von 0,0075 bis 0,5 Gew.-% und insbesondere von 0,01 bis 0,2 Gew.-%.

Es wurde gefunden, dass der zuvor genannte, synergistische Hautaufhellungseffekt besonders stark auftritt, wenn die Wirkstoffe a) und b) in einem bestimmten Gewichtsverhältnis in den kosmetischen Zusammensetzungen eingesetzt werden.

In einer vierten bevorzugten Ausführungsform enthalten die erfindungsgemäßen kosmetischen Zusammensetzungen die Komponenten a) und b) daher bevorzugt in einem Gewichtsverhältnis a) : b) von 20 : 1 bis 1 : 2, mehr bevorzugt von 17,5 bis 1 : 1, besonders bevorzugt von 15 : 1 bis 2 : 1, ganz besonders bevorzugt von 12,5 : 1 bis 5 : 1 und insbesondere von 10 : 1 bis 7,5 : 1.

Die erfindungsgemäßen Mittel weisen eine ausgezeichnete Hautverträglichkeit auf und vermögen pigmentierte Haut nicht nur aufzuhellen, sondern ihr zudem Glätte, Geschmeidigkeit und Weichheit zu verleihen.
Bei der Behandlung potentiell gereizter Haut wie beispielsweise der Haut nach der Rasur, unreiner und/oder mit Akne befallener Haut und/oder bei Vorliegen postinflammatorischer Hyperpigmentierungen kann es aber von Vorteil sein, wenn die erfindungsgemäßen kosmetischen Zusammensetzungen weiterhin einen Wirkstoff enthalten, der die Haut beruhigt.

Bevorzugte hautberuhigende Wirkstoffe, die in den erfindungsgemäßen kosmetischen Zusammensetzungen eingesetzt werden können, sind beispielsweise ausgewählt aus Farnesol, Allantoin, alpha-Bisabolol und/oder alpha-Liponsäure, besonders bevorzugt aus Allantoin und/oder alpha-Bisabolol und insbesondere bevorzugt aus Allantoin.
Der oder die hautberuhigenden Wirkstoffe können in den erfindungsgemäßen Mitteln bevorzugt in einer Menge von 0,01 bis 5 Gew.-%, mehr bevorzugt von 0,05 bis 3 Gew.-%, besonders bevorzugt von 0,075 bis 2 Gew.-% und insbesondere von 0,1 bis 1 Gew.-% eingesetzt werden, wobei sich die Mengenangaben auf das Gesamtgewicht des kosmetischen Mittels beziehen.

In einer weiteren bevorzugten Ausführungsform enthalten die erfindungsgemäßen kosmetischen Zusammensetzungen weiterhin Allantoin in einem Gewichtsanteil (bezogen auf das Gesamtgewicht der Zusammensetzung) von 0,01 bis 5 Gew.-%, bevorzugt von 0,05 bis 3 Gew.-% besonders bevorzugt von 0,075 bis 2 Gew.-% und insbesondere von 0,1 bis 1 Gew.-%.

Die erfindungsgemäßen Zubereitungen können prinzipiell in Form einer Lösung, einer Emulsion vom Typ Wasser-in-Öl (W/O) oder Öl-in-Wasser (O/W), einer multiplen Emulsion vom Typ Wasser-in-Öl-in- Wasser (W/O/W) oder Öl-in-Wasser-in-Öl (O/W/O), einer Hydrodispersion oder Lipodispersion, einem Gel, einem festen Stift und/oder einem Aerosol vorliegen.

Besonders bevorzugt liegen die erfindungsgemäßen Zusammensetzungen in Form einer Emulsion vor.

In einer weiteren bevorzugten Ausführungsform der Erfindung liegen die erfindungsgemäßen Zusammensetzungen in Form einer Lösung, einer Emulsion vom Typ Wasser-in-Öl (W/O) oder Öl-in-Wasser (O/W), einer multiplen Emulsion vom Typ Wasser-in-Öl-in-Wasser (W/O/W) oder Öl-in-Wasser-in-Öl (O/W/O), einer Hydrodispersion oder Lipodispersion, einem Gel, einem festen Stift und/oder einem Aerosol, besonders bevorzugt in Form einer Emulsion vor.

In einer insbesondere bevorzugten Ausführungsform liegen die erfindungsgemäßen Zusammensetzungen in Form einer Wasser-in-Öl-Emulsion (W/O-Emulsion) vor.

Emulsionen im Sinne der vorliegenden Erfindung können beispielsweise in Form von Cremes, Lotionen und/oder kosmetischen Milchen vorliegen und neben den zuvor genannten Wirkstoffen weiterhin bevorzugt Fette, Öle, Wachse und/oder andere Fettkörper sowie Wasser und einen oder mehrere Emulgatoren enthalten.

Die Ölphase liegt in den erfindungsgemäß bevorzugten Emulsionen vorzugsweise in einem Gewichtsanteil von 1 - 60 Gew.-% am Gesamtgewicht der Emulsion vor, besonders bevorzugt von 10 - 50 Gew.-% und außerordentlich bevorzugt von 15 - 35 Gew.-%.
Der oder die Emulgator(en) wird(werden) weder zur Ölphase noch zur Wasserphase gezählt. In einer bevorzugten Ausführungsform besteht die Ölphase zu mindestens 90 Gew.-% aus einer bei 20 °C flüssigen Ölkomponente.
Bevorzugte Ölkomponenten, die in den erfindungsgemäß bevorzugten Emulsionen eingesetzt werden können, sind beispielsweise ausgewählt aus:
- flüchtigen Silikonölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L 2), Octamethyltrisiloxan (L 3), Decamethyltetrasiloxan (L 4), beliebige Zweier- und Dreiermischungen aus L 2, L 3 und/oder L 4, wie sie z. B. in den Handelsprodukten DC 2-1184, Dow Corning® 200 (0,65 cSt) und Dow Corning® 200 (1,5 cSt) von Dow Corning enthalten sind,
- nichtflüchtigen höhermolekularen linearen Dimethylpolysiloxanen, im Handel erhältlich z. B. unter der Bezeichnung Dow Corning® 190, Dow Corning® 200 Fluid mit Viskositäten im Bereich von 5 - 100 cSt, bevorzugt 5 - 50 cSt oder auch 5 - 10 cSt, und Baysilon® 350 M,
- den Estern von linearen oder verzweigten gesättigten oder ungesättigten Fettalkoholen mit 2 bis 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Ethylhexylpalmitat (z. B. Cegesoft® C 24), Hexyldecylstearat (Eutanol® G 16), Hexyldecyllaurat, Isodecylneopentanoat, Isononylisononanoat, 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanot, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat,
- den Benzoesäureestern von linearen oder verzweigten C₈₋₂₂-Alkanolen, z. B. die Handelsprodukte Finsolv® TN (C₁₂-C₁₅-Alkylbenzoat), Finsolv® SB (Isostearylbenzoat) und Finsolv® EB (Ethylhexylbenzoat),
- den C₈-C₂₂-Fettalkoholestern einwertiger oder mehrwertiger C₂-C₇-Hydroxycarbonsäuren, insbesondere den Estern der Glycolsäure, Milchsäure, Äpfelsäure, Weinsäure, Citronensäure und Salicylsäure. Solche Ester auf Basis von linearen C_{12/15}-Alkanolen, z. B. C₁₂-C₁₅-Alkyllactat, und von in 2-Position verzweigten C_{12/13}-Alkanolen, z. B. Di-C₁₂-C₁₃-Alkylmalat, sind unter dem Warenzeichen Cosmacol® von der Firma Nordmann, Rassmann GmbH & Co, Hamburg, zu beziehen, insbesondere die Handelsprodukte Cosmacol® EMI, Cosmacol® ESI und Cosmacol® ETI,
- den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether (Ucon Fluid®, PPG-9-Butylether (Breox® B25), PPG-10-Butandiol (Macol® 57), PPG-3-Myristylether (Witconol® APM) und PPG-15-Stearylether (Arlamol® E),
- flüssigen Paraffinölen, Isoparaffinölen, z. B. die Handelsprodukte der Permethyl® -Serie, insbesondere Isododecan, Isohexadecan und Isoeicosan,
- synthetischen Kohlenwasserstoffen wie Polyisobuten oder Polydecen und alicyclischen Kohlenwasserstoffen, z. B. das Handelsprodukt 1,3-Di-(2-ethyl-hexyl)-cyclohexan (Cetiol® S),
- den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Besonders bevorzugte Alkoholöle sind beispielsweise Hexyldecanol (Eutanol® G), Octyldodecanol und 2-Ethylhexylalkohol,
- Mischungen aus Guerbetalkoholen und Guerbetalkoholestern, z. B. das Handelsprodukt Cetiol® PGL (Hexyldecanol und Hexyldecyllaurat),
- den symmetrischen, unsymmetrischen oder cyclischen Estern der Kohlensäure mit Fettalkoholen, beispielsweise Glycerincarbonat, Dicaprylylcarbonat (Cetiol® CC),
- Triglyceriden von linearen oder verzweigten, gesättigten oder ungesättigten, gegebenenfalls hydroxylierten C 8-30-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol® 318, Myritol® 331 (Cognis) oder Miglyol® 812 (Hüls) sowie Glyceryltriisostearin und die Handelsprodukte Estol® GTEH 3609 (Uniqema) oder Myritol® GTEH (Cognis),
- Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat,
- Di-n-alkylethern mit insgesamt 12 bis 36, insbesondere 12 bis 24 C-Atomen, z. B. Di-n-octylether (Cetiol® OE), Di-n-Hexyl-n-octylether und n-Octyl-n-decylether.

Besonders bevorzugte Öle sind die Silikonöle und die Ester von linearen oder verzweigten gesättigten oder ungesättigten Fettalkoholen mit 2 bis 30 Kohlenstoffatomen mit linearen oder verzweigten gesättigten oder ungesättigten Fettsäuren mit 2 - 30 Kohlenstoffatomen, vor allem Decamethylcyclopentasiloxan und Dodecamethylcyclohexasiloxan, Hexamethyldisiloxan (L 2), Octamethyltrisiloxan (L 3) und Decamethyltetrasiloxan (L 4) sowie beliebige Zweier- und Dreiermischungen aus L 2, L 3 und/oder L 4, flüchtige und nichtflüchtige lineare Silikonöle aus der Serie Dow Corning 200 Fluid mit Viskositäten von 0,65, 1,0, 1,5 und 5 cSt, die Esteröle 2-Ethylhexylpalmitat, Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv®, Estern von linearen oder verzweigten gesättigten oder ungesättigten Fettalkoholen mit 2 bis 30 Kohlenstoffatomen mit linearen oder verzweigten gesättigten oder ungesättigten Fettsäuren mit 2 - 30 Kohlenstoffatomen, C₁₂-C₁₅-Alkyllactat, Di-C₁₂-C₁₃-Alkylmalat, PPG-14-Butylether, die Handelsprodukte der Permethyl®-Serie, insbesondere Isododecan, Isohexadecan und Isoeicosan, sowie Polyisobuten und Polydecene sowie Mischungen der genannten Komponenten.

In einer weiteren bevorzugten Ausführungsform enthalten die erfindungsgemäßen kosmetischen Zusammensetzungen weiterhin mindestens ein Silikon in einem Gewichtsanteil (bezogen auf das Gesamtgewicht der Zusammensetzung) von 0,05 bis 20 Gew.-%, bevorzugt von 0,1 bis 17,5 Gew.-% besonders bevorzugt von 0,25 bis 15 Gew.-% und insbesondere von 0,5 bis 12,5 Gew.-%.

Es kann für einige Ausführungsformen der erfindungsgemäßen Zusammensetzungen ebenfalls bevorzugt sein, Mischungen der vorgenannten Öle einzusetzen. Dabei sind besonders Mischungen aus zwei Ölkomponententypen, z. B. einem flüchtigen Silikonöl und einem Esteröl, bevorzugt. Besonders bevorzugt sind Ölmischungen, die mindestens ein flüchtiges cyclisches und/oder lineares Silikonöl enthalten. Außerordentlich bevorzugt sind Ölmischungen, die überwiegend, das heißt zu mehr als 50 Gew.-%, bezogen auf die Ölmischung, mindestens ein flüchtiges cyclisches und/oder lineares Silikonöl enthalten. Weiterhin bevorzugt sind Ölmischungen, die 60 - 95 Gew.-%, besonders bevorzugt 70 - 90 Gew.-%, mindestens eines flüchtigen cyclischen und/oder linearen Silikonöls in Kombination mit 5 - 40 Gew.-%, besonders bevorzugt 10 - 30 Gew.-%, mindestens eines Esteröls, insbesondere eines der vorgenannten Esteröle, enthalten, jeweils bezogen auf das Gewicht der Ölmischung.

Die Wasserphase liegt in den erfindungsgemäß bevorzugten Emulsionen vorzugsweise in einem Gewichtsanteil von 40 - 99 Gew.-% am Gesamtgewicht der Emulsion vor, besonders bevorzugt von 50 - 90 Gew.-% und außerordentlich bevorzugt von 60 - 85 Gew.-%.

Zur Wasserphase zählen erfindungsgemäß bevorzugt Wasser sowie alle wasserlöslichen Inhaltsstoffe, mit Ausnahme der Emulgatoren. Bevorzugte kosmetische Zusammensetzungen in Form von Emulsionen sind durch einen Wassergehalt von 20 - 70 Gew.-%, bevorzugt 25 - 65 Gew.-% und besonders bevorzugt 30 - 60 Gew.-%, jeweils bezogen auf das Gesamtgewicht der Emulsion, gekennzeichnet.

Die erfindungsgemäß bevorzugten Emulsionen enthalten bevorzugt weiterhin mindestens einen Emulgator, bevorzugt einen Wasser-in-ÖI-Emulgator. Der mindestens eine Emulgator ist bevorzugt in einer Menge von 0,5 - 10 Gew.-%, besonders bevorzugt 1,0 - 7,5 Gew.-% und insbesondere von 1,5 - 5 Gew.-% in den Emulsionen enthalten, wobei sich die Mengenangaben auf das Gesamtgewicht der Emulsionen beziehen. Besonders bevorzugte Emulgatoren können ausgewählt sein aus:
- Poly-(C₂-C₃)alkylenglycol-modifizierten Silikonen mit den INCI-Bezeichnungen PEG-x Dimethicone (mit x = 2 - 20, bevorzugt 3-17, besonders bevorzugt 11-12), Bis-PEG-y Dimethicone (mit y = 3-25, bevorzugt 4 - 20), PEG/PPG a/b Dimethicone (wobei a und b unabhängig voneinander für Zahlen von 2- 30, bevorzugt 3-30 und besonders bevorzugt 12-20, insbesondere 14-18, stehen), Bis-PEG/PPG-c/d Dimethicone (wobei c und d unabhängig voneinander für Zahlen von 10-25, bevorzugt 14-20 und besonders bevorzugt 14-16, stehen) und Bis-PEG/PPG-e/f PEG/PPG g/h Dimethicone (wobei e, f, g und h unabhängig voneinander für Zahlen von 10-20, bevorzugt 14-18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440). Weitere erfindungsgemäß bevorzugte Emulgatoren sind Poly-(C₂-C₃)alkylenglycolmodifizierte Silikone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole und Alkyl Dimethicone Ethoxy Glucoside.
- Substanzen der allgemeinen Formel A-O-(CHR1-X-CHR2-O-)a-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe CHOR3 darstellt, R1 und R2 ein Wasserstoffatom oder eine Methylgruppe darstellen und so gewählt werden, dass nicht beide Reste gleichzeitig Methyl darstellen, und R3 ein Wasserstoffatom oder eine verzweigte oder unverzweigte, gesättigte oder ungesättigte Alkyl- oder Acylgruppe mit 1 bis 20 Kohlenstoffatomen darstellt.
- Besonders bevorzugt ist es, wenn die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H) b-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,
- gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden,
- ethoxylierten Alkoholen und Carbonsäuren mit 8 - 24 C-Atomen, insbesondere mit 16 - 22 C-Atomen, wie beispielsweise Steareth-2 oder Steareth-21,
- propoxylierten Alkoholen und Carbonsäuren mit 8 - 24 C-Atomen, insbesondere mit 16 - 22 C-Atomen,
- Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar,
- Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Veresterungsgrad von 1-10, vorzugsweise 1-5,
- Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1-10, vorzugsweise 1-5,
- Propylenglycolestern gesättigter und/oder ungesättigter, verzweigter und/oder unverzweigter Alkancarbonsäuren einer Kettenlänge von 8 - 24, insbesondere 12 - 18 C-Atomen,
- Methylglucose-Estern gesättigter und/oder ungesättigter, verzweigter und/oder unverzweigter Alkancarbonsäuren einer Kettenlänge von 8 - 24, insbesondere 12 - 18 C-Atomen,
- Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen. Es kann erfindungsgemäB von Vorteil sein, dass niedrig ethoxylierte (3 - 5 EO) und/oder propoxylierte Produkte Verwendung finden, beispielsweise polyethoxyliertes hydrogeniertes oder nichthydrogeniertes Ricinusöl oder ethoxyliertes Cholesterin.

Ganz besonders bevorzugte Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Batylalkohol, Polyethylenglycol(2)stearyl-ether (Steareth-2), Polyethylenglycol(21)stearyl-ether (Steareth-21), Glycerylsorbitanstearat, Polyglyceryl-4-lsostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyceryl-3-diisostearat, PEG-8 Distearat, Diglycerin Dipolyhydroxystearat, Glycerinisostearat, Sorbitanisostearat, Polyglyceryl-3-methylglucosedistearat, polyethoxyliertes hydrogeniertes oder nichthydrogeniertes Ricinusöl, ethoxyliertes Cholesterin, PEG-2 Stearat, PPG-15 Stearylether, PEG/PPG-18/18 Dimethicone, PEG-45/Dodecylglycolcopolymer, PEG-22/Dodecylglycolcopolymer und Methoxy PEG- 22/Dodecyl Glycol Copolymer. Insbesondere besonders bevorzugt ist es, wenn Kombinationen der oben genannten Emulgatoren, insbesondere eine Kombination aus zwei Emulgatoren, eingesetzt werden. Es kann erfindungsgemäß ebenfalls vorteilhaft sein, mindestens einen W/O-Emulgator in Kombination mit mindestens einem O/W-Emulgator einzusetzen.

Die erfindungsgemäß bevorzugten Emulsionen enthalten bevorzugt weiterhin Ethanol. Ethanol ist zum Beispiel bevorzugt, wenn der Frischeeffekt, der durch den hohen Wassergehalt der erfindungsgemäßen Zusammensetzungen hervorgerufen wird, weiter gesteigert werden soll.

Ethanol wird in den erfindungsgemäß bevorzugten Emulsionen bevorzugt in Mengen von 0 - 10 Gew.-%, besonders bevorzugt 0,01 - 7,5 Gew.-% und außerordentlich bevorzugt von 0,1 - 5 Gew.-% jeweils bezogen auf die gesamte Emulsion, eingesetzt.

Die erfindungsgemäß bevorzugten Emulsionen enthalten bevorzugt weiterhin mindestens ein wasserlösliches Polyol, ausgewählt aus den wasserlöslichen mehrwertigen C₂-C₉-Alkanolen mit 2 - 6 Hydroxylgruppen und wasserlöslichen Polyethylenglycolen mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 besonders bevorzugt sind. Auch Zucker und bestimmte Zuckerderivate wie Fructose, Glucose, Maltose, Maltitol, Mannit, Inosit, Sucrose, Trehalose und Xylose können erfindungsgemäB bevorzugt sein.
Besonders bevorzugt sind 1,2-Propylenglycol, Glycerin, 1,3-Butylenglycol, Diglycerin, Triglycerin, Dipropylenglycol und Tripropylenglycol.
Insbesondere bevorzugt sind 1,2-Propylenglycol und/oder Glycerin. Das wasserlösliche Polyol wird in den erfindungsgemäß bevorzugten Emulsionen bevorzugt in Mengen von 0,01 - 30 Gew.-%, besonders bevorzugt 0,1 - 27,5 Gew.-% und außerordentlich bevorzugt von 0,5 - 25 Gew.-% jeweils bezogen auf die gesamte Emulsion, eingesetzt.

Es wurde festgestellt, dass im axillaren Bereich durch häufige Rasuren entzündliche Reaktionen auf der Haut auftreten können, was unerwünschte Hautverfärbungen zur Folge haben kann. Um diesen Verfärbungen vorzubeugen bzw. um diese zu mindern oder zu beseitigen ist es in einer weiteren Ausführungsform von Vorteil, wenn die erfindungsgemäßen Zusammensetzungen als Deodorant-oder Antitranspirant-Zusammensetzung formuliert werden.

In einer fünften bevorzugten Ausführungsform enthalten die erfindungsgemäßen Zusammensetzungen daher weiterhin mindestens einen Antitranspirant-Wirkstoff in einem Gewichtsanteil (bezogen auf das Gesamtgewicht der Zusammensetzung) von 3 bis 40 Gew.-%, bevorzugt von 5 bis 35 Gew.-%, besonders bevorzugt von 7,5 bis 30 Gew.-% und insbesondere von 10 bis 25 Gew.-%.

Innerhalb dieser Ausführungsform enthalten die erfindungsgemäßen Zusammensetzungen mindestens einen wasserlöslichen Antitranspirant-Wirkstoff wie beispielsweise die wasserlöslichen adstringierenden anorganischen und organischen Salze des Aluminiums, Zirkoniums und Zinks bzw. beliebige Mischungen dieser Salze.
Besonders bevorzugte Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder-Polyethylenglykol (PEG), Aluminiumsesquichlorhydrex-PG oder-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Aluminiumundecylenoylkollagenaminosäure, Kaliumaluminiumsulfat, Natriumaluminiumlactat, Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat.

Unter "Wasserlöslichkeit" der Antitranspirant-Wirkstoffe wird eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Insbesondere bevorzugt sind adstringierende Aluminiumsalze, insbesondere Aluminiumchlorohydrat,
das beispielsweise pulverförmig als Micro Dry® (Reheis (Interorgana)), in Form einer wässrigen Lösung als Locron® L von Clariant, als Chlorhydrol® sowie in aktivierter Form als Reach® 103 oder AACH® 7172 von Summit vertrieben wird.
Unter der Bezeichnung Reach® 301 wird ein Aluminiumsesquichlorohydrat von Summit angeboten. Auch die Verwendung von Aluminium-Zirkonium-Trichlorohydrex-Glycin-Komplexen, die beispielsweise von Summit unter der Bezeichnung AAZG® 531 oder AAZG® 531D im Handel erhältlich sind, kann besonders vorteilhaft sein.
Die Antitranspirant-Wirkstoffe können auch als wässrige Lösungen eingesetzt werden.

Weitere bevorzugte kosmetische Wirkstoffe innerhalb dieser Ausführungsform sind Deodorant-Wirkstoffe, die besonders bevorzugt ausgewählt sind aus Geruchsabsorbern, deodorierend wirkenden Ionenaustauschern, keimhemmenden Mitteln, präbiotisch wirksamen Komponenten sowie Enzyminhibitoren oder, besonders bevorzugt, Kombinationen der genannten Wirkstoffe.

Silicate dienen als Geruchsabsorber, die gleichzeitig auch die rheologischen Eigenschaften der Zusammensetzungen innerhalb dieser Ausführunmgsform vorteilhaft unterstützen können. Zu den besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Als keimhemmende oder antimikrobielle Wirkstoffe bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid.
Des Weiteren sind Phenol, Arylalkohole wie insbesondere Phenoxyethanol, 2-Methyl-4-phenylbutan-2-ol und 2-Methyl-5-phenylpentan-1-ol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, alpha -Monoalkylglycerinether mit einem verzweigten oder linearen gesättigten oder ungesättigten, gegebenenfalls hydroxylierten C 6 - C 22-Alkylrest, besonders bevorzugt. alpha -(2-Ethylhexyl)glycerinether, im Handel erhältlich als Sensiva® SC 50 (ex Schülke & Mayr), Carbonsäureester, insbesondere Carbonsäuremonoester des Mono-, Di- und Triglycerins (insbesondere Glycerinmonolaurat, Diglycerinmonocaprinat, Diglycerinmonolaurat, Triglycerinmonolaurat und Triglycerinmonomyristat), Lantibiotika sowie Pflanzenextrakte (z. B. grüner Tee und Bestandteile des Lindenblütenöls) sind ebenfalls bevorzugt einsetzbar.
Weitere bevorzugte Deodorant-Wirkstoffe können ausgewählt sein aus sogenannten präbiotisch wirksamen Komponenten, worunter solche Komponenten zu verstehen sind, die nur oder zumindest überwiegend die geruchsbildenden Keime der Hautmikroflora hemmen, nicht aber die erwünschten, das heißt, die nicht-geruchsbildenden Keime. Explizit sind hier die Wirkstoffe, die in den Offenlegungsschriften DE 10333245 und DE 102004011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus Picea spp., Paullinia sp., Panax sp., Lamium album oder Ribes nigrum sowie Mischungen dieser Substanzen. Weitere bevorzugte Deodorant-Wirkstoffe können ausgewählt sein aus den keimhemmend wirkenden Parfümölen und den Deosafe-Parfümölen, die von der Firma Symrise erhältlich sind.

Zu den Enzyminhibitoren gehören Stoffe, die die für die Schweißzersetzung verantwortlichen Enzyme, insbesondere Arylsulfatase, beta -Glucuronidase, Aminoacylase, die esterspaltenden Lipasen und die Lipoxigenase, hemmen, z. B. Trialkylcitronensäureester, insbesondere Triethylcitrat, oder Zinkglycinat. Die Deodorant-Wirkstoffe können sowohl einzeln als auch in Mischungen eingesetzt werden. Besonders bevorzugt sind Phenoxyethanol, alpha -(2-Ethylhexyl)glycerinether, Diglycerinmonocaprinat, 2-Methyl-5-phenylpentan-1-ol, Mischungen aus Phenoxyethanol und alpha -(2-Ethylhexyl)glycerinether sowie Mischungen aus Arylalkoholen, insbesondere Phenoxyethanol, mit alpha -(2-Ethylhexyl)glycerinether und Diglycerinmonocaprinat, insbesondere die Mischungen aus alpha -(2-Ethylhexyl)glycerinether und 2-Methyl-5-phenylpentan-1-ol.

Die Gesamtmenge der Deodorant-Wirkstoffe in den Zusammensetzungen dieser Ausführungsform beträgt bevorzugt 0,01 - 10 Gew.-%, besonders bevorzugt 0,1 - 7 Gew.-%, insbesondere 0,2 - 5 Gew.-% und außerordentlich bevorzugt 0,3 - 1,0 Gew.-%, jeweils bezogen auf das Gesamtgewicht der Zusammensetzung.

Innerhalb dieser Ausführungsform kann es weiterhin von Vorteil sein, wenn die erfindungsgemäßen Zusammensetzungen zusätzlich mindestens ein Treibmittel und eine Aerosol-Abgabevorrichtung umfassen, wobei die mit der Zusammensetzung in Kontakt kommenden Teile des Ventils der Abgabevorrichtung bevorzugt aus nicht-metallischen Materialien bestehen.
Geeignete Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt.
Besonders bevorzugt sind Propan, n-Butan, iso-Butan sowie Mischungen dieser Treibgase. Insbesondere bevorzugt sind Propan/n-Butan-Mischungen im Gewichtsverhältnis von 10 : 90, bevorzugt von 20 : 80.
Die Zusammensetzungen dieser Ausführungsform und das (oder die) Treibmittel werden bevorzugt in einem Gewichtsverhältnis von 1 : 10 bis 2 : 1, bevorzugt von 1 : 7 bis 1 : 1 und insbesondere von 1 : 5 bis 1 : 2 in geeignete Aerosoldosen abgefüllt.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit.

In einer weiteren bevorzugten Ausführungsform können die erfindungsgemäßen Zusammensetzungen weiterhin mindestens eine Duftstoffkomponente enthalten. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Phenoxyethylisobutyrat, Benzylacetat, p-tert.-Butylcyclohexylacetat, Dimethylbenzylcarbinylacetat, Linalylacetat, Phenylethylacetat, Linalylbenzoat, Ethylmethylphenylglycinat, Benzylformiat, Allylcyclohexylpropionat, Styrallylpropionat und Benzylsalicylat. Zu den Ethern zählen zum Beispiel Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die lonone alpha -Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Geeignete Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl.
Die Duftstoffkomponente/n können in den erfindungsgemäßen Zusammensetzungen bevorzugt in Gesamtmengen von 0 bis 5 Gew.-%, besonders bevorzugt 0,1 - 4 Gew.-% und insbesondere von 0,5 - 3 Gew.-%, jeweils bezogen auf das Gewicht Zusammensetzungen, eingesetzt werden.

Ein zweiter Gegenstand der vorliegenden Erfindung ist die kosmetische, nicht-therapeutische Verwendung der erfindungsgemäßen kosmetischen Zusammensetzung zur Behandlung von Überpigmentierungen, Pigmentierungsstörungen, Altersflecken und/oder postinflammatorischen Hyperpigmentierungen der Haut.

Ein dritter Gegenstand der Erfindung ist ein Verfahren zur Herstellung kosmetischen Hautaufhellungs-Zusammensetzung in Form einer O/W- oder einer W/O-Emulsion, bei dem ein öliger Extrakt aus Liquorice-Wurzeln in der Wasserphase durch den Zusatz eines Ascorbylsäureesters gelöst wird.

Erfindungsgemäß besonders bevorzugte Verfahren sind dadurch gekennzeichnet, dass ein Isopropylmyristat-Extrakt aus Liquorice-Wurzeln in der Wasserphase mit Ascorbyltetraisopalmitat kombiniert wird.

Durch die Kombination wird der ölige Pflanzenextrakt vollständig in der Wasserphase der Emulsion gelöst.

### Beispiele:

### 1) Synergistischer Effekt von Süßholzextrakt (öllöslich) und Ascorbylsäureester

Der Einfluss von Süßholzextrakt (öllöslich) und Ascorbyltetraisopalmitat auf die Expression melanogeneserelevanter Gene wurde an rekonstruierten Melanozytenmodellen Typ VI der Firma Skinethic untersucht. Dabei wurden humane Keratinozyten zusammen mit TypVI Melanozyten kultiviert, so dass ein sehr dunkles Hautmodell entsteht. Zusammen bilden die Zellen ein dreidimensionales epitheliales Gewebe. Es wurden jeweils drei Modelle topisch mit 30 µl der Testformulierungen für 24 Stunden behandelt. Dabei wird die Formulierung zunächst auf das Modell gegeben und anschließend vorsichtig mit einem Pinsel verteilt. Die Inkubation findet anschließend bei 37°C mit 5% CO₂ statt. Als Kontrolle wurden unbehandelte Modelle sowie mit einer Leerkontrolle (3% PEG-40 Hydrogeniertes Rhizinusöl in Wasser) behandelte Modelle mitgeführt. Die Genexpressionen der melanozytenspezifischen Marker wurden mit Hilfe einer quantitativen Real-Time-PCR untersucht. Zur Durchführung der PCR wird zunächst mit Hilfe des RNeasy Mini Kits der Fa. Qiagen die RNA aus den Melanozytenmodellen isoliert und mittels reverser Transkription in cDNA umgeschrieben. Bei der anschließenden PCR Reaktion, die mit Hilfe genspezifischer Primer für die jeweiligen Marker durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und umso höher ist das Fluoreszenzsignal. Die Genexpression der zu bestimmenden Gene wird im Vergleich zur Leerkontrolle als verstärkte Expression mit Plus oder als verminderte Expression mit Minus gekennzeichnet (Tabelle 1).

Untersucht wurden verschiedene Melanogenese-assoziierte Parameter:
**ckit:** ckit ist der Rezeptor für den Wachstumsfaktor SCF. SCF wirkt über die Kopplung an den Rezeptor zellteilungsfördernd auf die Melanozyten. Wird die Rezeptorexpression verringert, kann die stimulierende Wirkung von SCF inhibiert werden.
**gp100:** gp100 codiert für ein Protein der der Melanosomenmembran, in dem das vonden Melanozyten produzierte Melanin zu den Keratinozyten transferiert wird. Durcheine Hemmung dieses Gens kann der Melanintransfer verringert werden.
**TRP1 und 2: TRP1** und 2 spielen als Enzyme eine wichtige Rolle beim enzymatischen Umbau von Tyrosin zu Melanin während der Melanin-Synthese.
**Tyr:** Tyrosinase ist das initial notwendige Enzym, das den ersten Schritt des Umbaus von Tyrosin zu Melanin katalysiert.

**Tabelle 1**

| | **ckit** | **Gp100** | **TRP1** | **TRP2** | **Tyr** |
|---|---|---|---|---|---|
| 0,5% Süßholzextrakt (öllöslich) | - | - | + | + | - |
| 0,05% Vitamin Tetraisopalmitat | 0 | - | - | + | + |
| 0,5% Süßholzextrakt (öllöslich) + 0,05% Ascorbyltetraisopalmitat | - | - | 0 | - | - |

Tabelle 1 zeigt die Genexpression unterschiedlicher Formulierungen. Die Substanzen wurden in Wasser, enthaltend 3 Gew.-% PEG-40 Hydrogenated Castor Oil, geprüft. Die Genexpression ist im Vergleich zur Leerkontrolle dargestellt.

Dabei steht:
- +: für eine verstärkte Expression
- -: für eine verminderte Expression
- 0: für keine Veränderung.

Die Daten zeigen, dass nur bei der Kombination der beiden Wirkstoffe Süßholzextrakt (öllöslich) und Ascorbyltetraisopalmitat eine zufriedenstellende und konsistente Genexpressionsänderung im Sinne einer verminderten Neubildung von Melanin eingetreten ist.

### 2) Unzureichende Wirkung von Süßholzextrakt (wasserlöslich)

In einem weiteren Versuch wurden Hautmodelle vom Typ SkinEthic (Fa. SkinEthic, France) enthaltend Keratinozyten und Melanozyten mit einer Formulierung enthaltend 1% eines wasserlöslichen Süßholzextrakts für 8 Tage inkubiert. Der Gehalt an Melanin wurde spektrophotochemisch bestimmt. Im Vergleich zur Leerkontrolle konnte keine Reduktion des Melaningehaltes festgestellt werden.

### 3) Ausführungsbesisoiele

### a) Antitranspirant-Roll-on (Mengenangaben in Gew.-%):

**Tabelle 2**

| | **A** | **B** | **C** | **D** |
|---|---|---|---|---|
| PPG-15 Stearyl Ether | 0,50 | 0,50 | 0,50 | 0,50 |
| Steareth-2 | 2,38 | 2,38 | 2,38 | 2,38 |
| Steareth-21 | 1,48 | 1,48 | 1,48 | 1,48 |
| Aluminium Chlorohydrate (50% in Wasser) | 40,00 | 40,00 | 40,00 | 40,00 |
| Talkum | 0,30 | 0,30 | 0,30 | 0,30 |
| Herbasol Extract Liquorice®¹ IPM | 0,50 | 1,00 | 2,00 | 0,50 |
| Nikkol®² VC IP | 0,05 | 0,10 | 0,20 | 0,05 |
| Allantoin | 0,10 | 0,30 | | 0,20 |
| Alpha-Bisabolol | | | 0,20 | |
| EDTA BX®³ Powder | 0,10 | 0,10 | 0,10 | 0,10 |
| Parfum | 0,65 | | 0,50 | |
| Wasser | ad 100 | ad 100 | ad 100 | ad 100 |

### Liste der verwendeten Rohstoffe:

- 1: INCI-Bezeichnung: Isopropyl Myristate, Glycyrrhiza Glabra (Licorice) Root Extract; Cosmetochem
- 2: INCI-Bezeichnung: Ascorbyl Tetraisopalmitate; Nikko Chemicals
- 3: INCI-Bezeichnung: Tetrasodium EDTA; BASF

### b) Antitranspirant Aerosol (Mengenangaben in Gew.-%):

**Tabelle 3**

| | **A** | **B** | **C** |
|---|---|---|---|
| Xiameter PMX-0245®⁴ | 10,70 | 10,70 | 10,70 |
| Dow Corning ES-5227®⁵ DM | 5,60 | 5,60 | 5,60 |
| Isopropylmyristat | 6,60 | 6,60 | 6,60 |
| Propylenglycol | 23,40 | 23,40 | 23,40 |
| Phenoxyethanol | 0,50 | 0,50 | 0,50 |
| Aluminium Chlorohydrate (50% in Wasser) | 50,00 | 50,00 | 50,00 |
| Herbasol Extract Liquorice®¹ IPM | 1,00 | 2,00 | 0,50 |
| Nikkol®² VC IP | 0,10 | 0,20 | 0,05 |
| Allantoin | 0,10 | | 0,20 |
| Alpha-Bisabolol | | 0,20 | |
| Parfum | 2,50 | 2,50 | 2,50 |
| Wasser | ad 100 | ad 100 | ad 100 |

Die Rezepturen A, B und C werden jeweils im Gewichtsverhältnis von 1 : 4 mit dem Treibmittel Propan/n-Butan (20/80) in Aerosoldosen abgefüllt.

### Liste der verwendeten Rohstoffe:

- 4: INCI-Bezeichnung: Cyclomethicone; Dow Corning
- 5: INCI-Bezeichnung: Dimethicone, PEG/PPG-18/18 Dimethicone; Dow Corning

Die Zusammensetzungen unter a) und b) weisen eine hervorragende Hautverträglichkeit auf. Sie sind stabil, zeigen keine Separationserscheinungen und führen zu glatter, gepflegter Haut. Nach mehrmaliger Anwendung konnte eine signifikante Aufhellung der behandelten Hautpartie festgestellt werden.

## Patentansprüche

1. Kosmetische, Antitranspirant-Zusammensetzung zur Hautaufhellung im axillaren Bereich, enthaltend
a) mindestens einen öligen Pflanzenextrakt aus der Gattung der Süßhölzer (*Glycyrrhiza*)*,*
b) mindestens einen Ascorbylsäureester und
c) mindestens einen Antitranspirant-Wirkstoff.

2. Kosmetische Antitranspirant-Zusammensetzung nach Anspruch 1, **dadurch gekennzeichnet, dass** sie einen öligen Extrakt aus den Wurzeln von *Glycyrrhiza Glabra* (Licorice-Wurzeln) enthält.

3. Kosmetische Antitranspirant-Zusammensetzung nach einem der vorherigen Ansprüche, **dadurch gekennzeichnet, dass** das Gewichtsverhältnis der Komponenten a) zu b) 20 : 1 bis 1 : 2, bevorzugt 17,5 : 1 bis 1 : 1, mehr bevorzugt 15 : 1 bis 2 : 1, besonders bevorzugt 12,5 : 1 bis 5 : 1 und insbesondere 10 : 1 bis 7,5 : 1 beträgt.

4. Kosmetische Antitranspirant-Zusammensetzung nach einem der vorherigen Ansprüche, **dadurch gekennzeichnet, dass** der Ascorbylsäureester ausgewählt ist aus Verbindungen der nachfolgenden Formel (I), 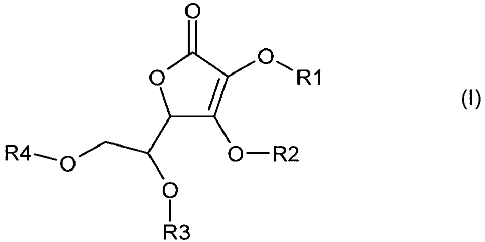 in der
einer bis vier der Reste R1 bis R4 für die Gruppierung -C(O)-R und die anderen Reste ggfs. für Wasserstoff stehen; und R für einen geradkettigen oder verzweigten, gesättigten oder ungesättigten Alk(en)ylrest mit 8 bis 24, bevorzugt mit 10 bis 20 und insbesondere mit 13 bis 17 Kohlenstoffatomen steht.

5. Kosmetische Antitranspirant-Zusammensetzung nach einem der vorherigen Ansprüche, **dadurch gekennzeichnet, dass** der Ascorbylsäureester gemäß Formel (I) ausgewählt ist aus Ascorbylpalmitat, Ascorbylisopalmitat, Ascorbyltetraisopalmitat, Ascorbylstearat, Ascorbylisostearat, Ascorbyloleat, und/oder Ascorbyllinoleat.

6. Kosmetische Antitranspirant-Zusammensetzung nach einem der vorherigen Ansprüche, **dadurch gekennzeichnet, dass** der Ascorbylsäureester gemäß Formel (I) Ascorbyltetraisopalmitat ist.

7. Kosmetische Antitranspirant-Zusammensetzung nach einem der vorherigen Ansprüche, **dadurch gekennzeichnet, dass** der Gewichtsanteil des mindestens einen Ascorbylsäureesters b) am Gesamtgewicht der Zusammensetzung 0,005 bis 1 Gew.-%, bevorzugt 0,0075 bis 0,5 Gew.-% und insbesondere 0,01 bis 0,2 Gew.-% beträgt.

8. Kosmetische Antitranspirant-Zusammensetzung nach einem der vorherigen Ansprüche, **dadurch gekennzeichnet, dass** sie einen Isopropylmyristat-Extrakt aus den Wurzeln von *Glycyrrhiza Glabra* (Licorice-Wurzeln) enthält.

9. Kosmetische Antitranspirant-Zusammensetzung nach einem der vorherigen Ansprüche, **dadurch gekennzeichnet, dass** sie weiterhin Allantoin in einem Gewichtsanteil (bezogen auf das Gesamtgewicht der Zusammensetzung) von 0,01 bis 5 Gew.-%, bevorzugt von 0,05 bis 3 Gew.-% besonders bevorzugt von 0,075 bis 2 Gew.-% und insbesondere von 0,1 bis 1 Gew.-% enthält.

10. Kosmetische Antitranspirant-Zusammensetzung nach einem der vorherigen Ansprüche, **dadurch gekennzeichnet, dass** sie weiterhin mindestens ein Silikon in einem Gewichtsanteil (bezogen auf das Gesamtgewicht der Zusammensetzung) von 0,05 bis 20 Gew.-%, bevorzugt von 0,1 bis 17,5 Gew.-% besonders bevorzugt von 0,25 bis 15 Gew.-% und insbesondere von 0,5 bis 12,5 Gew.-% enthält.

11. Kosmetische Antitranspirant-Zusammensetzung nach einem der vorherigen Ansprüche, **dadurch gekennzeichnet, dass** sie den Antitranspirant-Wirkstoff c) in einem Gewichtsanteil (bezogen auf das Gesamtgewicht der Zusammensetzung) von 3 bis 40 Gew.-%, bevorzugt von 5 bis 35 Gew.-%, besonders bevorzugt von 7,5 bis 30 Gew.-% und insbesondere von 10 bis 25 Gew.-% enthält.

12. Kosmetische Antitranspirant-Zusammensetzung nach einem der vorherigen Ansprüche, **dadurch gekennzeichnet, dass** sie in Form einer Lösung, einer Emulsion vom Typ Wasser-in-Öl (W/O) oder Öl-in-Wasser (O/W), einer multiplen Emulsion vom Typ Wasser-in-Öl-in-Wasser (W/O/W) oder Öl-in-Wasser-in-Öl (O/W/O), einer Hydrodispersion oder Lipodispersion, einem Gel, einem festen Stift und/oder einem Aerosol, vor allem in Form einer Emulsion, vorliegt.

13. Kosmetische, nicht-therapeutische Verwendung einer kosmetischen Antitranspirant-Zusammensetzung nach einem der vorherigen Ansprüche zur Behandlung von Überpigmentierungen, Pigmentierungsstörungen und/oder postinflammatorischen Hyperpigmentierungen im axillaren Bereich der Haut.

14. Verfahren zur Herstellung einer kosmetischen hautaufhellenden Antitranspirant-Zusammensetzungen in Form einer O/W- oder einer W/O-Emulsion, bei dem ein öliger Extrakt aus Liquorice-Wurzeln in der Wasserphase durch den Zusatz eines Ascorbylsäureesters gelöst wird.

15. Verfahren nach Anspruch 14, **dadurch gekennzeichnet, dass** ein Isopropylmyristat-Extrakt aus Liquorice-Wurzeln in der Wasserphase mit Ascorbyltetraisopalmitat kombiniert wird.

## Claims

1. A cosmetic antiperspirant composition for lightening skin in the armpit region, containing
a) at least one oily plant extract from the licorice genus (*Glycyrrhiza*)*,*
b) at least one ascorbyl acid ester, and
c) at least one antiperspirant active ingredient.

2. The cosmetic antiperspirant composition according to claim 1, **characterized in that** it contains an oily extract from the roots of *Glycyrrhiza glabra* (licorice roots).

3. The cosmetic antiperspirant composition according to one of the preceding claims, **characterized in that** the weight ratio of components a) to b) is from 20:1 to 1:2, preferably 17.5:1 to 1:1, more preferably 15:1 to 2:1, particularly preferably 12.5:1 to 5:1, and in particular 10:1 to 7.5:1.

4. The cosmetic antiperspirant composition according to one of the preceding claims, **characterized in that** the ascorbyl acid ester is selected from compounds of the following formula (I), 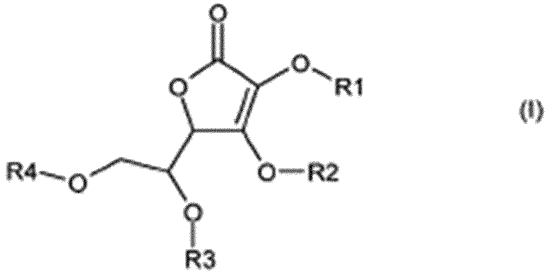 in which
one to four of the functional groups R1 to R4 represent the -C(O)-R group and the other functional groups possibly represent hydrogen; and R represents a straight-chain or branched, saturated or unsaturated alk(en)yl functional group having 8 to 24, preferably 10 to 20, and in particular 13 to 17, carbon atoms.

5. The cosmetic antiperspirant composition according to one of the preceding claims, **characterized in that** the ascorbyl acid ester according to formula (I) is selected from ascorbyl palmitate, ascorbyl isopalmitate, ascorbyl tetraisopalmitate, ascorbyl stearate, ascorbyl isostearate, ascorbyl oleate, and/or ascorbyl linoleate.

6. The cosmetic antiperspirant composition according to one of the preceding claims, **characterized in that** the ascorbyl acid ester according to formula (I) is ascorbyl tetraisopalmitate.

7. The cosmetic antiperspirant composition according to one of the preceding claims, **characterized in that** the weight proportion of the at least one ascorbyl acid ester b) in the total weight of the composition is from 0.005 to 1 wt.%, preferably 0.0075 to 0.5 wt.%, and in particular 0.01 to 0.2 wt.%.

8. The cosmetic antiperspirant composition according to one of the preceding claims, **characterized in that** it contains an isopropyl myristate extract from the roots of *Glycyrrhiza glabra* (licorice roots).

9. The cosmetic antiperspirant composition according to one of the preceding claims, **characterized in that** it also contains allantoin in a weight proportion (based on the total weight of the composition) of from 0.01 to 5 wt.%, preferably 0.05 to 3 wt.%, particularly preferably 0.075 to 2 wt.%, and in particular 0.1 to 1 wt.%.

10. The cosmetic antiperspirant composition according to one of the preceding claims, **characterized in that** it also contains at least one silicone in a weight proportion (based on the total weight of the composition) of from 0.05 to 20 wt.%, preferably from 0.1 to 17.5 wt.%, particularly preferably from 0.25 to 15 wt.%, and in particular from 0.5 to 12.5 wt.%.

11. The cosmetic antiperspirant composition according to one of the preceding claims, **characterized in that** it contains the antiperspirant active ingredient c) in a weight proportion (based on the total weight of the composition) of from 3 to 40 wt.%, preferably 5 to 35 wt.%, particularly preferably 7.5 to 30 wt.%, and in particular 10 to 25 wt.%.

12. The cosmetic antiperspirant composition according to one of the preceding claims, **characterized in that** it is in the form of a solution, an emulsion of the water-in-oil (W/O) type or oil-in-water (O/W) type, a multiple emulsion of the water-in-oil-in-water (W/O/W) type or the oil-in-water-in-oil (O/W/O) type, a hydrodispersion or lipodispersion, a gel, a solid stick and/or an aerosol, in particular in the form of an emulsion.

13. The cosmetic, non-therapeutic use of a cosmetic antiperspirant composition according to one of the preceding claims, for treating hyperpigmentation, pigmentation irregularities and/or post-inflammatory hyperpigmentation in the armpit region of the skin.

14. A method for preparing a cosmetic skin-lightening antiperspirant composition in the form of an O/W or W/O emulsion, wherein an oily extract from licorice roots is dissolved in the water phase by adding an ascorbyl acid ester.

15. The method according to claim 14, **characterized in that** an isopropyl myristate extract from licorice roots is combined with ascorbyl tetraisopalmitate in the water phase.

## Revendications

1. Composition antitranspirante cosmétique pour l'éclaircissement de la peau au niveau axillaire, contenant
a) au moins un extrait huileux de plantes du genre des réglisses (*Glycyrrhiza*)*,*
b) au moins un ester d'acide ascorbique et
c) au moins un principe actif antitranspirant.

2. Composition antitranspirante cosmétique selon la revendication 1, **caractérisée en ce qu'**elle contient un extrait huileux des racines de *Glycyrrhiza Glabra* (racines de réglisse).

3. Composition antitranspirante cosmétique selon l'une des revendications précédentes, **caractérisée en ce que** le rapport pondéral des composants a) sur b) s'élève de 20:1 à 1:2, de manière préférée de 17,5:1 à 1:1, de manière davantage préférée de 15:1 à 2:1, de manière particulièrement préférée de 12,5:1 à 5:1 et en particulier de 10:1 à 7,5:1.

4. Composition antitranspirante cosmétique selon l'une des revendications précédentes, **caractérisée en ce que** l'ester d'acide ascorbique est sélectionné parmi les composés de la formule (I) suivante, 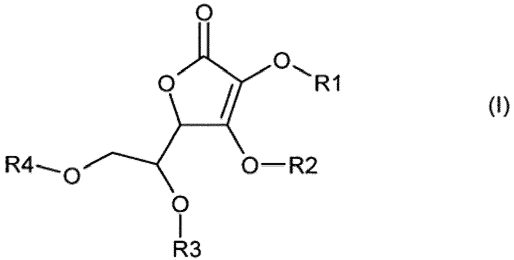 dans laquelle
un à quatre des résidus R1 à R4 représentent le groupement -C(O)-R et les autres résidus représentent le cas échéant de l'hydrogène ; et R représente un résidu alk(èn)yle à chaîne droite ou ramifié, saturé ou insaturé comportant 8 à 24, de manière préférée 10 à 20 et en particulier 13 à 17 atomes de carbone.

5. Composition antitranspirante cosmétique selon l'une des revendications précédentes, **caractérisée en ce que** l'ester d'acide ascorbique selon la formule (I) est sélectionné parmi le palmitate d'ascorbyle, l'isopalmitate d'ascorbyle, le tétraisopalmitate d'ascorbyle, le stéarate d'ascorbyle, l'isostéarate d'ascorbyle, l'oléate d'ascorbyle, et/ou le linoléate d'ascorbyle.

6. Composition antitranspirante cosmétique selon l'une des revendications précédentes, **caractérisée en ce que** l'ester d'acide ascorbique selon la formule (I) est le tétraisopalmitate d'ascorbyle.

7. Composition antitranspirante cosmétique selon l'une des revendications précédentes, **caractérisée en ce que** la part en poids de l'au moins un ester d'acide ascorbique b) dans le poids total de la composition vaut de 0,005 à 1 % en poids, de manière préférée de 0,0075 à 0,5 % en poids et en particulier de 0,01 à 0,2 % en poids.

8. Composition antitranspirante cosmétique selon l'une des revendications précédentes, **caractérisée en ce qu'**elle contient un extrait de myristate d'isopropyle issu des racines de *Glycyrrhiza Glabra* (racines de réglisse).

9. Composition antitranspirante cosmétique selon l'une des revendications précédentes, **caractérisée en ce qu'**elle contient en outre de l'allantoïne dans une proportion en poids (rapportée au poids total de la composition) de 0,01 à 5 % en poids, de manière préférée de 0,05 à 3 % en poids, de manière particulièrement préférée de 0,075 à 2 % en poids et en particulier de 0,1 à 1 % en poids.

10. Composition antitranspirante cosmétique selon l'une des revendications précédentes, **caractérisée en ce qu'**elle contient en outre au moins un silicone dans une proportion en poids (rapportée au poids total de la composition) de 0,05 à 20 % en poids, de manière préférée de 0,1 à 17,5 % en poids, de manière particulièrement préférée de 0,25 à 15 % en poids et en particulier de 0,5 à 12,5 % en poids.

11. Composition antitranspirante cosmétique selon l'une des revendications précédentes, **caractérisée en ce qu'**elle contient le principe actif antitranspirant c) dans une proportion en poids (rapportée au poids total de la composition) de 3 à 40 % en poids, de manière préférée de 5 à 35 % en poids, de manière particulièrement préférée de 7,5 à 30 % en poids et en particulier de 10 à 25 % en poids.

12. Composition antitranspirante cosmétique selon l'une des revendications précédentes, **caractérisée en ce qu'**elle se présente sous la forme d'une solution, d'une émulsion du type eau-dans-huile (E/H) ou huile-dans-eau (H/E), d'une émulsion multiple du type eau-dans-huile-dans-eau (E/H/E) ou huile-dans-eau-dans-huile (H/E/H), d'une hydrodispersion ou d'une lipodispersion, d'un gel, d'un bâton solide et/ou d'un aérosol, majoritairement sous la forme d'une émulsion.

13. Utilisation cosmétique, non thérapeutique, d'une composition antitranspirante cosmétique selon l'une des revendications précédentes pour le traitement de surpigmentations, de troubles de la pigmentation et/ou d'hyperpigmentations post-inflammatoires au niveau axillaire de la peau.

14. Procédé de production d'une composition antitranspirante éclaircissant la peau sous la forme d'une émulsion H/E ou d'une émulsion E/H, dans laquelle un extrait huileux de racines de réglisse est dissous dans la phase aqueuse par l'ajout d'un ester d'acide ascorbique.

15. Procédé selon la revendication 14, **caractérisé en ce qu'**un extrait de myristate d'isopropyle de racines de réglisse est combiné avec du tétraisopalmitate d'ascorbyle dans la phase aqueuse.
